# EUROPÄISCHE PATENTSCHRIFT

(11) **EP 3 778 027 B1**
(45) Veröffentlichungstag und Bekanntmachung des Hinweises auf die Patenterteilung: **20.04.2022**
(21) Anmeldenummer: 20189386.4
(22) Anmeldetag: 04.08.2020
(51) Int. Cl.: B01L 3/00, C12M 1/26, C12M 1/00, G01N 33/49

(54) **TRENNKÖRPER UND VERFAHREN ZUM TRENNEN VON BLUTPLASMA UND BLUTZELLEN**
SEPARATING BODY AND METHOD FOR SEPARATING BLOOD PLASMA AND BLOOD CELLS
SÉPARATEUR ET PROCÉDÉ DE SÉPARATION DU PLASMA SANGUIN ET DES CELLULES SANGUINES

(30) Priorität: 13.08.2019 DE 102019121723
(43) Veröffentlichungstag der Anmeldung: 17.02.2021
(73) Patentinhaber: Sarstedt AG & Co. KG, 51588 Nümbrecht (DE)
(72) Erfinder: WEINSTOCK, Mark, 57612 Helmenzen (DE)
(74) Vertreter: Grosse, Wolf-Dietrich Rüdiger

(56) Entgegenhaltungen:
- EP-A2- 0 311 011
- DE-A1- 2 819 170
- US-A1- 2010 288 694

## Beschreibung

Die Erfindung betrifft einen Trennkörper sowie ein Verfahren zum Trennen von Blutzellen und Blutplasma in einem Blutentnahmeröhrchen. Darüber hinaus betrifft die Erfindung ein Blutentnahmeröhrchen mit dem besagten Trennkörper sowie ein Verfahren zum Herstellen des Trennkörpers.

Trennkörper für Blutentnahmeröhrchen sowie entsprechende Blutentnahmeröhrchen sind im Stand der Technik grundsätzlich bekannt, so z. B. aus der deutschen Offenlegungsschrift DE 28 19 170 A1, der US-Anmeldung US 2010/0288694 A1, aus der internationalen Patentanmeldung WO 2010/132783 A1 oder aus dem europäischen Patent EP 0311011 B1.

Die deutsche Offenlegungsschrift DE 28 19 170 A1 offenbart ein Verfahren und eine Vorrichtung zum Herstellen eines Ventils in z.B. einem Blutentnahmeröhrchen. Der dort offenbarte Trennkörper umfasst einen Schwimmkörper mit einem umlaufenden Dichtrand zur dichtenden Anlage an der Innenseite des Blutentnahmeröhrchens. Der Schwimmkörper umfasst weiterhin eine Durchgangsöffnung, die mithilfe eines Ventilkörpers verschließbar ist. Der Ventilkörper ist über elastische Stränge an den Innenwänden des zylindrischen Schwimmkörpers befestigt.

Die US-Anmeldung 2010/0288694 A1 offenbart einen elastischen Trennkörper mit einem Schwimmkörper und einem Ballastkörper zur Separation von zwei Phasen von Blut in z.B. einem Blutentnahmeröhrchen. Der Schwimmkörper weist einen umlaufenden Dichtrand auf zur dichtenden Anlage an den Innenseiten des Blutentnahmeröhrchens. Der Trennkörper und insbesondere dessen Schwimmkörper weist keine Durchgangsöffnung auf und folglich weist er auch kein Ventil auf zum Verschließen oder Öffnen dieser Durchgangsöffnung.

Die in der WO- und der EP-Schrift offenbarten Trennkörper bestehen aus einem Schwimmkörper mit einer ersten Dichte und mindestens einer Durchgangsöffnung sowie einem Ballastkörper mit einer zweiten Dichte, welche größer ist als die erste Dichte des Schwimmkörpers. Die Gesamtdichte des Trennkörpers, d. h. des Schwimmkörpers und des Ballastkörpers zusammen liegt zwischen der Dichte des Plasmas und der Zellen in dem Blut. Der Schwimmkörper und der Ballastkörper gemäß dem EP-Patent EP 0311011 B1 sind relativ zueinander bewegbar ausgebildet und bilden zusammen ein Ventil. Zur Realisierung einer Ventilfunktion in dem Trennkörper sind der Schwimmkörper und der Ballastkörper jeweils mit wulstartigen Rändern ausgebildet. Zum Öffnen des Ventils für eine Flüssigkeit, z. B. Blut, bewegen sich die einander gegenüberliegenden wulstartigen Ränder des Schwimmkörpers und des Ballastkörpers voneinander weg und sind beabstandet voneinander; zum Verschließen des Ventils bewegen sich die wulstartigen Ränder aufeinander zu und liegen dann dichtend aneinander. An seiner der Durchgangsöffnung in dem Schwimmkörper zugewandten Unterseite weist der Ballastkörper mindestens eine Rinne bzw. mindestens einen Kanal auf, um einen Durchtritt der Flüssigkeit durch die Durchgangsöffnung auch dann sicherzustellen, wenn der Ballastkörper z. B. aufgrund der Einwirkung einer Zentrifugalkraft mit seiner Unterseite gegen den Schwimmkörper drückt.

Der technischen Lehre der EP 0 311 011 B1 haften folgende Nachteile an: Die Herstellbarkeit von dieser technischen Lehre erscheint nur unter hohem Aufwand möglich zu sein. Es scheint nur eine manuelle Montage der Komponenten nach Bereitstellung der entsprechenden Einzelteile in Frage zu kommen. Weiter ist die Ausbildung der wulstartigen Ränder sowohl an dem Schwimmkörper wie auch an dem Ballastkörper aufwändig und relativ teuer. Der Flüssigkeitsaustausch durch die engen Spalte zwischen den Rändern erhöht das Risiko einer Hämolyse und ggf. einer längeren Separationsdauer. Gerade die Hämolyse führt zur Verunreinigung vom Blutplasma, welches bei der Separation gewonnen werden soll. Schließlich erscheint die Funktionalität sehr theoretisch. Gerade der Ansatz, dass die unterschiedlichen Drücke innerhalb der aufgezeigten Kammern Einfluss auf die Ventilstellung haben, geht mit hoher Wahrscheinlichkeit mit Füllproblemen, langen Füllzeiten und ggf. auch mit Problemen in Bezug auf die Präparationskonzentration einher. Konkret führen Zellablagerung im flach ausgebildeten Bereich des Ballastkörpers von EP0311011 B1 zu schlechterer Probenqualität.

Der Erfindung liegt die Aufgabe zugrunde, einen alternativen Trennkörper für ein Blutentnahmeröhrchen, ein Blutentnahmeröhrchen mit dem alternativen Trennkörper, ein alternatives Verfahren zum Trennen von Blutzellen und Blutplasma sowie ein alternatives Verfahren zum Herstellen des Trennkörpers bereitzustellen.

Diese Aufgabe wird bezüglich des Trennkörpers durch den Gegenstand des Patentanspruchs 1 gelöst. Demnach weist der Ballastkörper zur Ausbildung des Ventils weiterhin mindestens ein Verschlussmittel auf zum Öffnen oder Verschließen der Durchgangsöffnung in dem Schwimmkörper.

Der Ausdruck "Durchgangsöffnung" meint ein Loch oder eine Durchgangsbohrung in dem Schwimmköper zum Durchtritt einer Flüssigkeit von einer auf die gegenüberliegende andere Seite des Schwimmkörpers. Die Durchgangsöffnung ist erfindungsgemäß so groß ausgebildet, dass sie in geöffnetem Zustand einen problemlosen Austausch der Flüssigkeit zwischen den Kammern oberhalb und unterhalb des Trennkörpers in dem Blutentnahmeröhrchen ermöglicht. Auf diese Weise wird das Risiko der Hämolyse und ggf. einer längeren Separationsdauer reduziert.

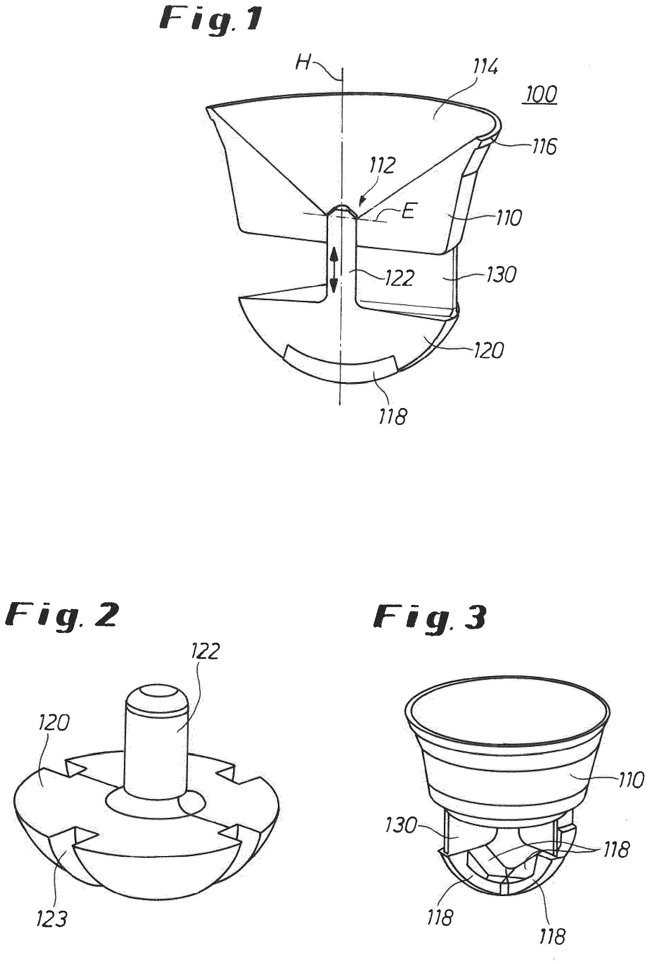

Die Begriffe "oben, oberhalb, unten, unterhalb, vertikal und horizontal" beziehen sich auf die insbesondere in Figur 1 gezeigte Ausrichtung des Trennkörpers im Raum.

Weil bei dem erfindungsgemäßen Trennkörper der Ballastkörper unterhalb des Schwimmkörpers angeordnet ist, öffnet das Ventil, während sich der Trennkörper aufgrund von einwirkender Zentrifugalkraft von seiner Ausgangsstellung an die Phasengrenze zwischen dem Blutplasma und den Blutzellen bewegt. Während dieser Zeit besteht die Möglichkeit, dass Luftbläschen, welche zunächst unterhalb des Trennkörpers in dem Blutentnahmeröhrchen vorhanden sind, bis zum Verschließen der Durchgangsöffnung immer noch durch die geöffnete Durchgangsöffnung hindurch in den oberen Teil des entnommenen Blutes in dem Blutentnahmeröhrchen, d. h. in das Plasma übergehen und von dort in den Bereich oberhalb der Phasengrenze entweichen können. Zusätzlich können die Luftbläschen auch aufgrund eines sehr elastisch ausgebildeten Dichtrandes des Schwimmkörpers zwischen dem Dichtrand und der Wandung das Probenröhrchen aus dem Bereich unterhalb des Trennkörpers in den Bereich oberhalb des Trennkörpers emporsteigen. Beide Möglichkeiten bieten den Vorteil, dass der Trennkörper nicht durch die Auftriebskraft der Luftbläschen unterhalb des Trennkörpers daran gehindert wird, auf die Phasengrenze abzusinken. Außerdem wird auf diese Weise eine schiefe Anordnung des Trennkörpers innerhalb des Blutentnahmeröhrchens in der Grenzschicht vermieden. Stattdessen richtet sich der Trennkörper in der Grenzschicht grade, d. h. symmetrisch zur Längsachse des Blutentnahmeröhrchens aus.

Die Durchgangsöffnung in dem Schwimmkörper soll in Abhängigkeit des Betrages einer auf den Ballastkörper und den Schwimmkörper einwirkenden Zentrifugalkraft mit dem Ballastkörper verschließbar oder zu öffnen sein. Das Zusammenspiel zwischen der Dichte der Flüssigkeitsprobe in dem Blutentnahmeröhrchen sowie des Schwimmkörpers und des Ballastkörpers bewirkt die Öffnung des Ventils. Zu diesem Zweck sieht die Erfindung erfindungsgemäß ein Rückstellelement vor zum federnden Verbinden des Schwimmkörper mit dem Ballastkörper so, dass zum Öffnen des Ventils ein Kraftaufwand erforderlich ist zum Überwinden einer durch das Rückstellelement definierten Rückstellkraft. Der erforderliche Kraftaufwand wird bei der vorgesehenen Anwendung des Trennkörpers, nämlich zum Trennen von Plasma bzw. Serum und Zellen im Blut, durch Einwirken der Zentrifugalkräfte aufgebracht.

Der Schwimmkörper ist vorteilhafterweise gemäß einem weiteren Ausführungsbeispiel in Form eines Trichters ausgebildet. Diese Ausbildung des Trennkörpers bietet - je nach Steilheit der Trichteröffnung - den Vorteil, dass nur noch sehr wenige, im Idealfall keine Zellen aus dem Blut mehr auf der Oberfläche des Schwimmkörpers haften bleiben. Stattdessen wandern unter Einfluss der Zentrifugalkraft vorzugsweise alle Zellen durch die Durchgangsöffnung in dem Schwimmkörper in den Bereich unterhalb des Trennkörpers in dem Blutentnahmeröhrchen. Auf diese Weise wird die Qualität der später zu analysierenden Flüssigkeits- bzw. Blutprobe, die insbesondere aus der Flüssigkeit oberhalb des Trennkörpers besteht, deutlich verbessert. Der Ballastkörper ist unterhalb des Schwimmkörpers, insbesondere außerhalb des Trichters angeordnet.

Die Durchgangsöffnung in dem Schwimmkörper ist vorzugsweise so ausgerichtet, dass das Lot auf die von ihr aufgespannte Ebene mit der Hauptachse des Trennkörpers zusammenfällt, d. h. dass der Winkel zwischen dem besagten Lot und der Hauptachse des Trennkörpers 0° beträgt. Dies ist jedoch keine zwingend notwendige Ausgestaltung: Vielmehr kann ein grundsätzlich beliebiger Winkel α zwischen dem Lot und der Hauptachse bestehen; Voraussetzung ist lediglich, dass die Durchgangsöffnung in dem Schwimmkörper durch den Ballastkörper verschließbar ist. Insofern sind auch die beanspruchten Winkel von α = +/- 45° bzw. α = +/- 10° lediglich beispielhaft und keineswegs singulär beschränkend zu verstehen. Weitere Ausgestaltungen des Trennkörpers, insbesondere betreffend die Ausbildung des Verschlussmittels an dem Ballastkörper zum Verschließen der Durchgangsöffnung in dem Schwimmkörper, die Ausbildung des Rückstellelementes, die Ausbildung des Trichters, betreffend das Material des Schwimmkörpers sowie betreffend die Halterung des Ballastkörpers an dem Schwimmkörper sind Gegenstand der abhängigen Ansprüche zu dem beanspruchten Trennkörper.

Die oben genannte Aufgabe wird weiterhin durch ein Blutentnahmeröhrchen mit dem erfindungsgemäßen Trennkörper gelöst. Dieses ist dadurch gekennzeichnet, dass der durch den umlaufenden Dichtrand gebildete maximale Außendurchmesser des Schwimmkörpers größer ist als der Innendurchmesser des Blutentnahmeröhrchens für eine umlaufend dichtende Anlage des Dichtrandes an den Innenseiten der Wände des Blutentnahmeröhrchens.

Die oben genannte Aufgabe wird weiterhin gelöst durch ein Verfahren nach Anspruch 10 Die Vorteile dieses Verfahrens entsprechen den oben mit Bezug auf den Trennkörper genannten Vorteilen. Nach einer Ausgestaltung des beanspruchten Verfahrens wandert der Trennkörper nach seinem Lösen aus der Ausgangsstellung A - unter Überwindung der Haftreibung zwischen dem Dichtrand des Trennkörpers und den Innenwänden des Blutentnahmeröhrchens durch die Zentrifugalkraft - in die Grenzschicht zwischen dem Plasma und den Zellen des aufgetrennten Blutes. Nach der axialen Ausrichtung des Trennkörpers liegt dessen umlaufender Dichtrand vollumfänglich dichtend an der Innenseite des Blutentnahmeröhrchens an. Das Ausrichten des Ventils in vertikaler Richtung setzt den Beginn einer Zentrifugation voraus. Bei Einwirkung einer steigenden Zentrifugalkraft auf den Trennkörper sinkt dieser in Richtung der Phasengrenze zwischen den zu trennenden Bestandteilen der Flüssigkeit ab, bei Blut zwischen das Plasma und die Blutzellen. Das Ventil wird nach der axialen Ausrichtung des Trennkörpers in der Grenzschicht nach dem Wegfall der Zentrifugalkraft aufgrund der wirkenden Rückstellkraft eines Rückstellelementes wieder verschlossen.

Der Begriff "axiale Ausrichtung" bedeutet in diesem Zusammenhang, dass der Trennkörper innerhalb des Blutentnahmeröhrchens in der Grenzschicht so angeordnet ist, dass seine Hauptachse idealerweise mit der Längsachse des Blutentnahmeröhrchens zusammenfällt. Kleinere Winkelabweichungen zwischen der Hauptachse und der Längsachse sind von dem Begriff "axiale Ausrichtung" eingeschlossen; Voraussetzung ist jedoch auf jeden Fall, dass auch bei einer Schiefstellung des Trennkörpers der umlaufende Dichtrand des Schwimmkörpers immer noch umlaufend dichtend an den Innenseiten der Wände des Blutentnahmeröhrchens anliegen muss.

Die Begriffe "Phasengrenze" und "Grenzschicht" werden synonym verwendet. Beide Begriffe meinen den Übergang zwischen den Flüssigkeitskomponenten unterschiedlicher Dichte oberhalb und unterhalb des Trennkörpers. Die aufzutrennenden Flüssigkeitskomponenten, beispielsweise Blutzellen und Blutplasma, haben unterschiedliche Dichten. Die Dichte des Trennkörpers ist so gewählt, dass sie zwischen den Dichten der beiden Flüssigkeitskomponenten liegt. Dadurch wird erreicht, dass sich der Trennkörper bei einer Zentrifugation des Blutentnahmeröhrchens mit der Flüssigkeit genau zwischen die beiden aufzutrennenden Flüssigkeitskomponenten, d. h. an die Phasengrenze setzt / bewegt.

Der Begriff "Hauptachse des Trennkörpers" meint die Achse in vertikaler Richtung durch den Schwimmkörper und den Ballastkörper, wie sie insbesondere in der Figur 1 gezeigt ist.

Für die Herstellung des Trennkörpers bietet sich insbesondere das Zwei-Komponenten-Spritzguss-Verfahren an, wobei der Schwimmkörper die eine Komponente und der Ballastkörper die andere Komponente bildet. Wichtig ist, dass die beiden Komponenten aus unterschiedlichen Materialien spritzgegossen werden, welche keine chemische oder stoffliche Verbindung miteinander eingehen und auch keine Adhäsion zueinander haben. Dies ist deshalb wichtig, weil der Schwimmkörper und der Ballastkörper völlig unabhängig voneinander bzw. relativ zueinander beweglich bleiben müssen, ohne dass sie aneinander "kleben" bleiben. Insbesondere dieses Zwei-Komponenten-Spritzguss-Verfahren bietet den Vorteil, dass manuelle Montagearbeiten weitgehend entfallen und deshalb der erfindungsgemäße Trennkörper sehr kostengünstig und mit nur relativ geringem Aufwand hergestellt werden kann. Alternativ können der Schwimmkörper und der Ballastkörper jeweils auch unabhängig voneinander z. B. im Spritzgussverfahren hergestellt und anschließend zusammengefügt werden. Weiterhin besteht die Möglichkeit, zuerst den Ballastkörper herzustellen, diesen dann in ein weiteres Spritzgießwerkzeug zu legen und den Schwimmkörper aufzuspritzen, so dass der Ballastkörper umspritzt wird.

Weitere vorteilhafte Ausgestaltungen der Erfindung sind Gegenstand der abhängigen Ansprüche.

Der Beschreibung sind insgesamt 13 Figuren beigefügt, wobei
- Figur 1: ein erstes Ausführungsbeispiel für den erfindungsgemäßen Trennkörper;
- Figur 2: den Ballastkörper des Trennkörpers nach Figur 1 als Einzelteil;
- Figur 3: den Schwimmkörper des Trennkörpers nach Figur 1 als Einzelteil;
- Figur 4: ein zweites Ausführungsbeispiel für den erfindungsgemäßen Trennkörper in einem Querschnitt;
- Figur 5: ein drittes Ausführungsbeispiel für den erfindungsgemäßen Trennkörper in einem Querschnitt;
- Figur 6: ein viertes Ausführungsbeispiel für den erfindungsgemäßen Trennkörper in einem Querschnitt;
- Figur 7: ein Blutentnahmeröhrchen in einer Querschnittsdarstellung mit dem erfindungsgemäßen Trennkörper in einer Ausgangsposition;
- Figur 8: den erfindungsgemäßen Trennkörper in der Ausgangsposition nach Figur 7 in einer Draufsicht;
- Figur 9: das Blutentnahmeröhrchen mit dem Trennkörper in verschiedenen Stellungen während einer erfolgenden Zentrifugation;
- Figur 10: den Trennkörper in dem Blutentnahmeröhrchen in der Endstellung in einer Grenzschicht zwischen Blutplasma und Blutzellen mit noch geöffnetem Ventil;
- Figur 11: den Trennkörper in der Endstellung nach Figur 10; diesmal allerdings mit geschlossenem Ventil;
- Figur 12: eine erste Möglichkeit zur Entnahme von Blutplasma aus dem Blutentnahmeröhrchen nach erfolgter Zentrifugation; und
- Figur 13: eine zweite Möglichkeit zum Entnehmen der Blutplasma aus dem Blutentnahmeröhrchen nach der Zentrifugation zeigt.

Die Erfindung wird nachfolgend unter Bezugnahme auf die genannten Figuren in Form von Ausführungsbeispielen detailliert beschrieben. In allen Figuren sind gleiche technische Elemente mit gleichen Bezugszeichen bezeichnet.

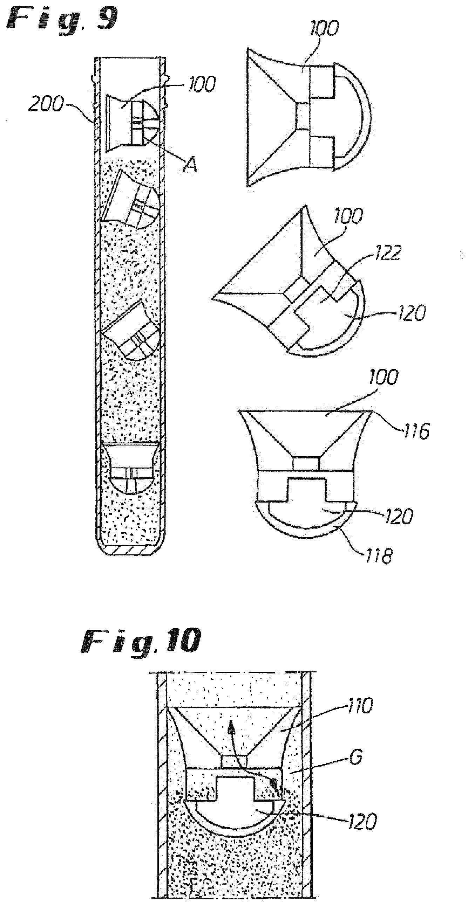

Figur 1 zeigt den erfindungsgemäßen Trennkörper 100 zur Verwendung in einem Blutentnahmeröhrchen 200, siehe beispielsweise Figur 9. Das Blutentnahmeröhrchen dient zur Aufnahme von Blut, welches einem Patienten entnommen wurde. Der Trennkörper 100 in dem Blutentnahmeröhrchen dient zum Trennen von Blutplasma und Blutzellen in dem Blut während einer Zentrifugation des Blutentnahmeröhrchens. Der besagte Zentrifugationsprozess ist wesentlicher Bestandteil in der Präanalytik von Blut zu medizinischen Zwecken. Der Trennkörper 100 weist einen Schwimmkörper 110 mit einer ersten Dichte und mindestens einer Durchgangsöffnung 112 auf. An dem Schwimmkörper ist mindestens ein umlaufender Dichtrand 116 ausgebildet zur umlaufend dichtenden Anlage an der Innenseite des Blutentnahmeröhrchens. Neben dem Schwimmkörper weist der Trennkörper 100 einen Ballastkörper 120 auf mit einer zweiten Dichte, welche größer als die erste Dichte des Schwimmkörpers 110 ist. Die Gesamtdichte des Trennkörpers, d. h. des Schwimmkörpers und des Ballastkörpers zusammen betrachtet, liegt zwischen der Dichte des Plasmas und der Dichte der Zellen in dem Blut.

Erfindungsgemäß bilden der Schwimmkörper 110 und der Ballastkörper 120 zusammen ein Ventil zum Öffnen oder Verschließen der Durchgangsöffnung 112 in dem Schwimmkörper 110. Zu diesem Zweck sind der Schwimmkörper 110 und der Ballastkörper 120 in Richtung des Doppelpfeiles relativ zueinander bewegbar angeordnet.

Das Rückstellelement 130 realisiert eine federnde Verbindung zwischen dem Schwimmkörper 110 und dem Ballastkörper 120 so, dass zum Öffnen des Ventils ein Kraftaufwand erforderlich ist, weil eine durch das Rückstellelement 130 definierte Rückstellkraft überwunden werden muss. Das Rückstellelement kann sowohl mit dem Schwimmkörper als auch mit dem Ballastkörper verbunden sein. Dies ist jedoch bei der in den Figuren 1 bis 3 gezeigten Ausgestaltung nicht erforderlich; dort wird die elastische Verbindung dadurch realisiert, dass das Rückstellelement eine federnde Verbindung zwischen dem Schwimmkörper und dem Haltebügel 118 bildet und der Ballastkörper 120 von dem Haltebügel 118 getragen wird. Das Rückstellelement 130 kann aus demselben Material wie der Schwimmkörper 110 und optional auch aus demselben Material wie der Haltebügel 118 bestehen; vorzugsweise ist es einstückig mit dem Schwimmkörper und dem Haltebügel 118 ausgebildet. Alternativ kann das Rückstellelement auch aus demselben Material wie der Ballastkörper und vorzugsweise einstückig mit diesem ausgebildet sein.

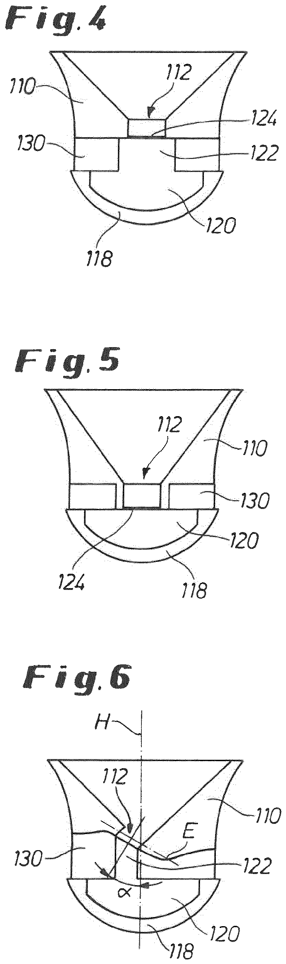

Zur Ausbildung des Ventils ist die mindestens eine Durchgangsöffnung 112 in dem Schwimmkörper 110 in Richtung auf den Ballastkörper 120 hin geöffnet. Bei dem in Figur 1 gezeigten Ausführungsbeispiel steht die durch die Durchgangsöffnung 112 aufgespannte Ebene E senkrecht zu der Hauptachse H des Trennkörpers 100. Anders ausgedrückt fällt das Lot auf die Ebene E mit der Hauptachse H zusammen bzw. ist parallel zu der Hauptachse H ausgerichtet. Diese Ausgestaltung der Durchgangsöffnung 112 ist jedoch keineswegs zwingend, wie weiter unten auch in Figur 6 gezeigt wird.

Zur Realisierung des erfindungsgemäßen Ventils ist es weiterhin erforderlich, dass der Ballastkörper 120 mindestens ein Verschlussmittel 122 aufweist zum Öffnen oder Verschließen der Durchgangsöffnung 112 in dem Schwimmkörper 110. Dieses Verschlussmittel ist in Figur 1 beispielsweise in Form eines Dorns 122 ausgebildet, welcher in die Durchgangsöffnung 112 in dem Schwimmkörper 110 einführbar ist, um diesen abdichtend zu verschließen.

Der Schwimmkörper 110 ist vorzugsweise in Form eines Trichters ausgebildet. Der Trichter verjüngt sich ausgehend von einem oberen Durchgangsöffnungsquerschnitt 114 zu der mindestens einen Durchgangsöffnung 112 hin und mündet in diese Durchgangsöffnung 112. Beispielhaft ist der umlaufende Dichtrand 116 an dem oberen Durchgangsöffnungsquerschnitt 114 ausgebildet zur umlaufend dichtenden Anlage an den Innenseiten der Wände des Blutentnahmeröhrchens 200. Der obere große Durchgangsöffnungsquerschnitt 114 ist bezogen auf die vertikale Ausrichtung des Trennkörpers, wie in Figur 1 gezeigt, oberhalb der Durchgangsöffnung 112 angeordnet.

Figur 2 zeigt den Ballastkörper 120 mit dem besagten Dorn 122 in einer Einzeldarstellung.

Figur 3 zeigt den Schwimmkörper 110, wie er auch in Figur 1 gezeigt ist, in Einzeldarstellung. Zu erkennen ist, dass an der Unterseite des Schwimmkörpers Rückstellelemente 130 angeordnet sind, welche Haltebügel 118 tragen, welche zur Aufnahme und zum Halten des Ballastkörpers 120 dienen. Der Schwimmkörper 110, die Rückstellelemente 130 und die Haltebügel 118 können aus demselben Material gebildet sein und sind vorzugsweise auch einstückig zusammen ausgebildet. Die in Figur 2 am Rand des Ballastkörpers 120 erkennbar ausgebildeten Nuten 123 dienen zur Aufnahme der in Figur 3 gezeigten Haltebügel 118. Zusammengesetzt ergeben der Ballastkörper 120 nach Figur 2 und der Schwimmkörper 110 mit dem Rückstellelement 130 und dem Haltebügel 118 nach Figur 3 den in Figur 1 gezeigten erfindungsgemäßen Trennkörper 100.

Figur 4 zeigt ein zweites Ausführungsbeispiel für den erfindungsgemäßen Trennkörper 100 in einem Querschnitt, wobei sich dieses Ausführungsbeispiel dadurch auszeichnet, dass der Dorn 122 des Ballastkörpers 120 hier größer als der Durchgangsöffnungsquerschnitt der Durchgangsöffnung 112 ausgebildet ist, so dass der Dorn 122 nicht in die Durchgangsöffnung 112 eindringen kann. Das Verschließen der Durchgangsöffnung 112 wird in diesem Ausführungsbeispiel dadurch realisiert, dass das Verschlusselement 124 in Form einer Anlagefläche als Flächenbereich an der Stirnseite des Dornes 122 ausgebildet ist. Die Anlagefläche deckt die Durchgangsöffnung 112 in dem Schwimmkörper dichtend ab.

Figur 5 zeigt ein drittes Ausführungsbeispiel für den erfindungsgemäßen Trennkörper 100. Bei dieser Ausgestaltung mündet die Durchgangsöffnung 112 des Schwimmkörpers 110 in einen auf den Ballastkörper hin ausgerichteten kurzen Kanal. Das ballastkörperseitige Ende des Kanals, und damit die Durchgangsöffnung 112 wird hier ebenfalls durch eine Anlagefläche 124 als Flächenbereich auf den Ballastkörper 120 dichtend abgedeckt und damit verschlossen. Ein Dorn 122 ist in diesem Ausführungsbeispiel an dem Ballastkörper 120 entbehrlich.

Figur 6 zeigt ein viertes Ausführungsbeispiel für den erfindungsgemäßen Trennkörper, bei welchem die Durchgangsöffnung 112 unter einem Winkel α gegenüber der Hauptachse H des Trennkörpers geneigt ausgebildet ist. Die durch die Durchgangsöffnung 112 aufgespannte Ebene E bildet mit ihrem Lot den Winkel α zur Hauptachse H. Wichtig ist bei diesem wie bei allen anderen Ausführungsbeispielen der vorliegenden Erfindung, dass der Ballastkörper bzw. dessen Verschlussmittel so ausgebildet ist, dass er auch in diesem Fall die Durchgangsöffnung 112 dichtend abdeckt. Bei dem in Figur 6 gezeigten fünften Ausführungsbeispiel kann das dadurch erfolgen, dass der Dorn 122 an seiner oberen Stirnseite entsprechend der Durchgangsöffnung 112 abgeschrägt und eine zusätzlich dichtende Anlagefläche aufweist. Der Winkel α kann grundsätzlich beliebige Werte zwischen 0° ≤ α < 90° annehmen; er beträgt jedoch vorzugsweise 0°.

Nachfolgend wird das erfindungsgemäße Verfahren zum Auftrennen von Blut in Plasma und Zellen in einem Blutentnahmeröhrchen 200 mit Hilfe des erfindungsgemäßen Trennkörpers näher beschrieben:

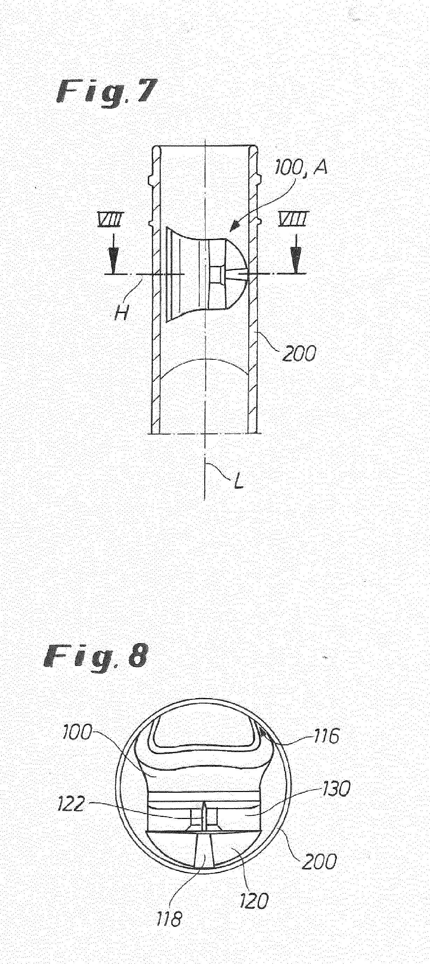

Figur 7 zeigt das Blutentnahmeröhrchen 200 mit dem darin angeordneten erfindungsgemäßen Trennkörper 100 in einem Auslieferungszustand bzw. einem Ausgangszustand bzw. in einer Ausgangsstellung A. Dabei ist das Blutentnahmeröhrchen mit einer Schraubkappe verschlossen. Im Auslieferungszustand A befindet sich der Trennkörper um ca. 90° verdreht zur Längsachse L des Blutentnahmeröhrchens. Somit ist sichergestellt, dass eine entnommene Blutprobe an dem Trennkörper 100 vorbei in den unteren Bereich des Blutentnahmeröhrchens fließen kann. Dabei ist das Ventil innerhalb des Trennkörpers 100 in verschlossener Position (Figur 7).

Das Blutentnahmeröhrchen wird über seine Verschlusskappe, beispielsweise eine Schraubkappe mit Blut gefüllt.

Figur 8 zeigt den Trennkörper 100 positioniert in seiner Ausgangsstellung A innerhalb des Blutentnahmeröhrchens 200 in einer Draufsicht. In dieser Stellung ist der Dichtrand 116 zwangsweise stark verformt; deshalb ist es wichtig, dass der Schwimmkörper aus elastischem Material gefertigt ist.

Das Blut läuft in dieser Ausgangsstellung A an dem Trennkörper, respektive an der inneren Wand des Blutentnahmeröhrchens vorbei. Dabei ist, wie gesagt, das Ventil geschlossen, so dass kein Blut durch den Trennkörper hindurch fließen kann.

Figur 9 zeigt einen Querschnitt durch das Blutentnahmeröhrchen 200 mit dem Trennkörper in verschiedenen Lagen bzw. Stellungen unter Einfluss einer Zentrifugation. Das Blutentnahmeröhrchen wird zentrifugiert, um das darin enthaltene von einem Patienten entnommene Blut zu zentrifugieren und es auf diese Weise in das besagte Blutplasma und die besagten Blutzellen aufzutrennen. Durch die Zentrifugation schwenkt der Trennkörper 100 aus seiner Ausgangsstellung A aus. Durch die immer größere werdende Zentrifugalkraft auf den Trennkörper und die elastische Anbindung des Ballastkörpers 120 an den Schwimmkörper 110 öffnet sich das Ventil, so dass nunmehr auch ein Austausch von Flüssigkeit durch den Trennkörper 100 hindurch möglich ist. Konkret wandern die Zellen in dem Blut aufgrund der Zentrifugalkraft durch die Durchgangsöffnung 112 in dem Trennkörper 100 hindurch in den unteren Teil des Blutentnahmeröhrchens, weil die Zellen schwerer sind als das besagte Blutplasma. Das Blutplasma tritt nicht durch die Durchgangsöffnung 112 hindurch, sondern verbleibt stattdessen in dem oberen Teil des Blutentnahmeröhrchens oberhalb des Trennkörpers 100. Durch die Zentrifugation wird der Trennkörper 100 in dem Blutentnahmeröhrchen auf das Niveau, d. h. den Höhenbereich der Phasengrenze zwischen den schwereren Zellen und dem leichteren Blutplasma bewegt, um diese beiden Komponenten voneinander zu trennen. Wenn sich der Trennkörper 100 in dieser Phasengrenze, auch Grenzschicht G genannt, befindet, liegt der Dichtrand 116 umlaufend dichtend an den Innenseiten der Wände des Blutentnahmeröhrchens an, weil sein Durchmesser in entspanntem Zustand größer ist als der Innendurchmesser des Blutentnahmeröhrchens.

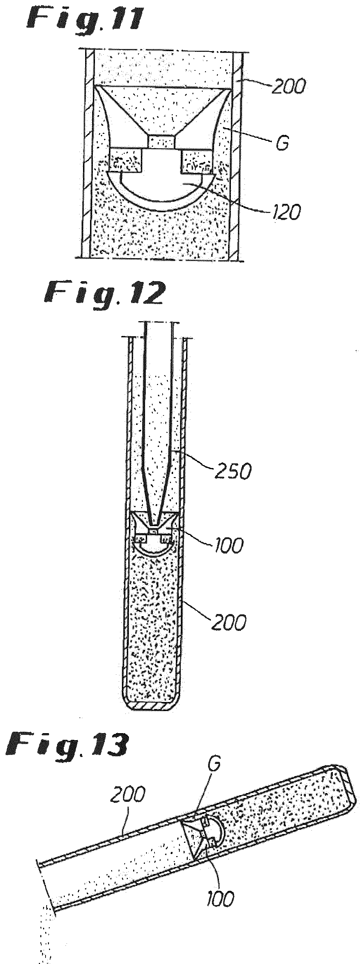

In der Phasengrenze bzw. Grenzschicht G ist das Ventil bzw. die Durchgangsöffnung 112 im Schwimmkörper 110 gemäß Figur 10 zunächst noch geöffnet. Solange die Zentrifugation noch anhält, ermöglicht das geöffnete Ventil eine Kavitäten-Kommunikation, d. h. einen barrierefreien Austausch von einzelnen Bestandteilen des Blutes zwischen dem Bereich oberhalb und unterhalb des Trennkörpers 100 in dem Blutentnahmeröhrchen und somit die besagte Phasentrennung. Nach erfolgter Phasentrennung wird die Zentrifugation gestoppt. Durch die fehlende Zentrifugalkraft wird der Ballastkörper dann aufgrund der von dem Rückstellelement 130 ausgeübten Rückstellkraft auf den Schwimmkörper 110 zu bewegt und die Durchgangsöffnung 112 wird verschlossen; siehe Figur 11. Die obere und untere Kavität, d. h. der Bereich oberhalb und unterhalb des Trennkörpers 100 in dem Blutentnahmeröhrchen, sind jetzt durch den Passsitz der Dichtlippe 116 zu den Innenseiten der Wände des Blutentnahmeröhrchens und durch das geschlossene Ventil im Trennkörper dicht voneinander getrennt. Damit sind auch das Blutplasma und die Zellen, wie gewünscht, wirksam voneinander getrennt.

Das für eine Analyse des Blutes besonders wichtige Blutplasma kann nun beispielsweise, wie in Figur 12 gezeigt, mit Hilfe einer Pipettenspitze 250 abpipettiert werden. Hier bietet die trichterförmige Ausgestaltung des Schwimmkörpers den besonderen Vorteil, dass die Pipettenspitze bis in den Bereich der Durchgangsöffnung 112 hineinragen kann und somit auch einen letzten verbleibenden Rest von Blutplasma aus der oberen Kavität entnehmen kann.

Schließlich zeigt Figur 13 die Möglichkeit, das Blutplasma durch Kippen des Blutentnahmeröhrchens abzugießen; dies ist insbesondere möglich durch den vollumfänglich abdichtenden Passsitz des Trennkörpers 100 mit seinen Dichtlippen 116 im Inneren des Blutentnahmeröhrchens und das geschlossene Ventil.

### Bezugszeichenliste

- 100: Trennkörper
- 110: Schwimmkörper
- 112: Durchgangsöffnung in den Schwimmkörper
- 114: Durchgangsöffnungsquerschnitt
- 116: Dichtrand
- 118: Haltebügel
- 120: Ballastkörper
- 122: Verschlussmittel, z.B. Dorn
- 123: Nuten in dem Ballastkörper
- 124: Verschlussmittel
- 130: Rückstellelement
- 200: Blutentnahmeröhrchen
- 250: Pipettenspitze

- d: Innendurchmesser des Blutentnahmeröhrchens
- A: Ausgangsstellung
- E: von der Durchgangsöffnung aufgespannte Ebene
- G: Grenzschicht
- H: Hauptachse des Trennkörpers
- L: Längsachse des Blutentnahmeröhrchens
- α: Winkel

## Patentansprüche

1. Trennkörper (100), zum Trennen von Blutplasma und Blutzellen in einem Blutentnahmeröhrchen (200), wobei der Trennkörper aufweist:
einen Schwimmkörper (110) mit einer ersten Dichte, und mit mindestens einem umlaufenden Dichtrand (116); und
einen Ballastkörper (120) mit einer zweiten Dichte, welche größer als die erste Dichte ist;
wobei die Gesamtdichte des Schwimmkörpers und des Ballastkörpers zwischen der Dichte des Plasmas und der Dichte der Zellen in dem Blut liegt;
wobei der Schwimmkörper (110) und der Ballastkörper (120) relativ zueinander bewegbar sind und zusammen ein Ventil bilden;
**dadurch gekennzeichnet,**
**dass** der Schwimmkörper mindestens eine Durchgangsöffnung (112) aufweist;
**dass** der Dichtrand (116) des Schwimmkörpers (110) ausgebildet ist zur umlaufend dichtenden Anlage an der Innenseite des Blutentnahmeröhrchens;
**dass** zur Ausbildung des Ventils weiterhin der Ballastkörper (120) mindestens ein Verschlussmittel (122, 124) aufweist zum Öffnen oder Verschließen der Durchgangsöffnung (112) in dem Schwimmkörper (110); und
**dass** ein Rückstellelement (130) vorgesehen ist als federnde Verbindung zwischen dem Schwimmkörper (110) und dem Ballastkörper (120) so, dass zum Öffnen des Ventils ein Kraftaufwand zum Überwinden einer durch das Rückstellelement (130) definierten Rückstellkraft erforderlich ist.

2. Trennkörper (100) nach Anspruch 1,
**dadurch gekennzeichnet,**
**dass** das Verschlussmittel (122) in Form eines Dorns ausgebildet ist, welcher in die Durchgangsöffnung in dem Schwimmkörper (110) einführbar ist.

3. Trennkörper (100) nach Anspruch 1,
**dadurch gekennzeichnet,**
**dass** das Verschlussmittel (124) in Form einer Anlagefläche als Flächenbereich auf dem Ballastkörper (120), ausgebildet ist zum dichtenden Abdecken der Durchgangsöffnung (112) in dem Schwimmkörper (110).

4. Trennkörper (100) nach Anspruch 1,
**dadurch gekennzeichnet,**
**dass** das Rückstellelement (130) aus demselben Material wie der Schwimmkörper (110), vorzugsweise einstückig mit diesem, oder aus demselben Material wie der Ballastkörper (120), vorzugsweise einstückig mit diesem gebildet ist.

5. Trennkörper (100) nach einem der vorangegangenen Ansprüche,
**dadurch gekennzeichnet,**
**dass** der Schwimmkörper (110) in Form eines Trichters ausgebildet ist, wobei sich der Trichter ausgehend von einem Durchgangsöffnungsquerschnitt (114), an welchem der vorzugsweise umlaufende Dichtrand (116) zur umlaufend dichtenden Anlage an den Innenseiten der Wände des Blutentnahmeröhrchens (200) ausgebildet ist, zu der mindestens einen Durchgangsöffnung (112) hin verjüngt und in die Durchgangsöffnung mündet; und
wobei der Durchgangsöffnungsquerschnitt - bezogen auf eine vertikale Ausrichtung des Trennkörpers - oberhalb der Durchgangsöffnung (112) angeordnet ist.

6. Trennkörper (100) nach einem der vorangegangenen Ansprüche,
**dadurch gekennzeichnet,**
**dass** der Schwimmkörper (110), insbesondere im Bereich seines Dichtrandes (116) aus elastischem Material gefertigt ist.

7. Trennkörper (100) nach einem der vorangegangenen Ansprüche,
**dadurch gekennzeichnet,**
**dass** die Durchgangsöffnung (112) in dem Schwimmkörper derart ausgerichtet ist, dass das Lot auf die von ihr aufgespannte Ebene mit der Hauptachse (H) des Trennkörpers einen Winkel von α = 45°, vorzugsweise von α = 10°, weiter vorzugsweise von α = 0° aufweist.

8. Trennkörper (100) nach einem der vorangegangenen Ansprüche,
**dadurch gekennzeichnet,**
**dass** der Schwimmkörper (110) mindestens einen Haltebügel (118) aufweist zum Halten und/oder Führen des Ballastkörpers (120), insbesondere bei seiner Bewegung relativ zu dem Schwimmkörper (110).

9. Blutentnahmeröhrchen (200) mit einem Trennkörper (100) in seinem Inneren, **dadurch gekennzeichnet,**
**dass** der Trennkörper (100) ausgebildet ist nach einem der vorangegangenen Ansprüche; und
**dass** der durch den umlaufenden Dichtrand (116) gebildete maximale Außendurchmesser des Schwimmkörpers (110) in entspanntem Zustand größer ist als der Innendurchmesser (d) des Blutentnahmeröhrchens (200) für eine umlaufend dichtende Anlage an den Innenseiten der Wände des Blutentnahmeröhrchens.

10. Verfahren zum Trennen von Blut in Plasma und Zellen in einem verschlossenen Blutentnahmeröhrchen (200) mit Hilfe eines Trennkörpers (100) im Inneren des Blutentnahmeröhrchens nach einem der vorangegangenen Ansprüche, mit folgenden Schritten:
- Einbringen von Blut in das Röhrchen (200), wobei das Blut an dem querliegenden Trennkörper außen vorbei in das Innere des Röhrchens fließt;
- Zentrifugieren des Röhrchens mit dem darin enthaltenen Blut, wobei sich das Blutplasma und Blutzellen auftrennt, und wobei sich der Trennkörper (100) aus einer Ausgangsstellung (A) löst, in eine axiale Ausrichtung zur Längsachse (L) des Blutentnahmeröhrchens (200) übergeht und in eine Grenzschicht (G) zwischen dem aufgetrennten Plasma und den Zellen wandert;
wobei das Ventil in dem Trennkörper (100) in der Ausgangsstellung (A) geschlossen ist; und
wobei sich das Ventil unter Einwirkung der Zentrifugalkraft entgegen einer Rückstellkraft öffnet, so dass die im Vergleich zu dem Plasma schwereren Zellen des Blutes während des Zentrifugierens unter Einwirkung der Zentrifugalkraft durch die geöffnete Durchgangsöffnung (112) in dem Schwimmkörper (110) hindurch treten und sich unterhalb des Trennkörpers (100) in dem Blutentnahmeröhrchen (200) ansammeln, während das leichtere Plasma oberhalb des Trennkörpers in dem Blutentnahmeröhrchen (200) verbleibt.

11. Verfahren nach Anspruch 10,
**dadurch gekennzeichnet,**
**dass** der Trennkörper (100) nach seinem Lösen aus der Ausgangsstellung (A) - unter Überwindung der Haftreibung durch die Zentrifugalkraft - in die Grenzschicht (G) wandert; und
**dass** der umlaufende Dichtrand (116) des Trennkörpers (100) nach der axialen Ausrichtung des Trennkörpers mit dem Ventil reibschlüssig und vollumfänglich dichtend an der Innenseite des Blutentnahmeröhrchens (200) anliegt; und
**dass** das sich das Ventil nach seiner axialen Ausrichtung in der Grenzschicht (G) - nach Wegfall der Zentrifugalkraft - aufgrund einer wirkenden Rückstellkraft wieder schließt.

12. Verfahren nach Anspruch 11,
**dadurch gekennzeichnet,**
**dass** im Anschluss an die axiale Ausrichtung des Trennkörpers in der Grenzschicht (G) und nach dem Schließen des Ventils das Plasma aus dem Blutentnahmeröhrchen entnommen wird, beispielsweise durch Abpipettieren mit Hilfe einer Pipette oder durch Abkippen.

13. Verfahren nach einem der Ansprüche 10 bis 12,
**dadurch gekennzeichnet,**
**dass** der Trennkörper (100) vor dem Einbringen von Blut in dem Blutentnahmeröhrchen in der Ausgangsstellung (A) vorinstalliert ist, wobei der Trennkörper (100) mit seiner Hauptachse (H) quer zur Längsrichtung (L) des Blutentnahmeröhrchens ausgerichtet ist.

## Claims

1. Separator (100) for separating blood plasma and blood cells in a blood sampling tube (200), wherein the separator comprises:
a floating element (110) with a first density and with at least one circumferential sealing edge (116); and
a ballast element (120) with a second density which is greater than the first density;
wherein the total density of the floating element and of the ballast element lies between the density of the plasma and the density of the cells in the blood;
wherein the floating element (110) and the ballast element (120) are movable relative to each other and together form a valve;
**characterised in that**
the floating element comprises at least one through opening (112);
**in that** the sealing edge (116) of the floating element (110) is configured for circumferential sealing application on the inner side of the blood sampling tube;
**in that** to form the valve, the ballast element (120) further comprises at least one closing means (122, 124) for opening or closing the through opening (112) in the floating element (110); and
**in that** a return element (130) is provided as a springloaded connection between the floating element (110) and the ballast element (120) so that for opening the valve, an exertion of force is required in order to overcome a restoring force defined by the return element (130) .

2. Separator (100) according to claim 1,
**characterised in that**
the closure means (122) is designed in the form of a mandrel which can be introduced into the through opening in the floating element (110).

3. Separator (100) according to claim 1,
**characterised in that**
that the closing means (124) in the form of a contact surface, is designed as a flat area on the ballast element (120) for covering of the through opening (112) in the floating element (110) in a sealing manner.

4. Separator (100) according to claim 1,
**characterised in that**
the return element (130) is made of the same material as the floating element (110), and preferably in one piece therewith, or of the same material as the ballast element (120) and preferably in one piece therewith.

5. Separator (100) according to any one of the preceding claims,
**characterised in that**
the floating element (110) is designed in the form of funnel, wherein, starting from a through opening cross-section (114) on which the preferably circumferential sealing edge (116) for circumferential sealing application to the inner sides of the walls of the blood sampling tube (200) is formed, the funnel tapers towards the at least one through opening (112) and opens into the through opening; and
wherein the through opening cross section - in relation to a vertical alignment of the separator - is arranged above the through opening (112).

6. Separator (100) according to any one of the preceding claims,
**characterised in that**
the floating element (110), particularly in the area of its sealing edge (116), is made of elastic material.

7. Separator (100) according to any one of the preceding claims,
**characterised in that**
the through opening (112) is orientated in the floating body in such a way that the perpendicular to the plane formed by it with the main axis (H) of the separator comprises an angle of α = 45°, preferably of α = 10°, more preferably of α = 0°.

8. Separator (100) according to any one of the preceding claims,
**characterised in that**
the floating element (110) comprises at least one holding bracket (118) for holding and/or guiding the ballast element (120), more particularly during its movement relative to the floating element (110).

9. Blood sampling tube (200) with a separator (100) in its interior,
**characterised in that**
the separator (100) is designed in accordance with any one of the preceding claims; and
**in that** in the relaxed state, the maximum outer diameter of the floating element (110) formed by the circumferential edge (116) is greater than the inner diameter (d) of the blood sampling tube (200) for application to the inner sides of the walls of the blood sampling tube in a circumferentially sealing manner.

10. Method of separating blood into plasma and cells in a closed blood sampling tube (200) by way of a separator (100) in the interior of the blood sampling tube according to any one of the preceding claims, with the follow steps:
- introduction of blood into the tube (200), wherein the blood flows externally past the transverse separator into the interior of the tube;
- centrifugation of the tube with the blood contained therein, wherein the blood plasma and blood cells separate, and wherein the separator (100) leaves an initial position (A), becomes axially aligned to the longitudinal axis (L) of the blood sampling tube (200) and moves into a boundary layer (G) between the separated plasma and the cells;
wherein the valve in the separator (100) is closed in the initial position (A); and
wherein through the effect of the centrifugal force, the valve opens contrary to a restoring force so that due to the effect of the centrifugal force during centrifugation, the cells of the blood, which are heavier in comparison to the plasma, pass through the opened through opening (112) in the floating element (110) and collect below the separator (100) in the blood sampling tube (200), while the lighter plasma remains above the separator in the blood sampling tube (200).

11. Method according to claim 10,
**characterised in that**
after leaving the initial position (A) - overcoming the static friction through the centrifugal force - the separator (100) moves into the boundary layer (G); and
**in that** that after the axial alignment of the separator with the valve, the circumferential edge (116) of the separator (100) is in frictional sealing contact on the inner side of the blood sampling tube (200) over its full extent; and
**in that** after its axial alignment in the boundary layer (G) - following discontinuation of the centrifugal force - the valve closes again through the effect of a restoring force.

12. Method according to claim 11,
**characterised in that**
following the axial alignment of the separator in the boundary layer (G) and after closure of the valve, the plasma is removed from the blood sampling tube, for example by way of pipetting using a pipette or through decanting.

13. Method according to any one of claims 10 to 12, **characterised in that**
before introducing blood into the blood sampling tube, the separator (100) is pre-installed in the initial position (A), wherein the separator (100) is aligned with its main axis (H) perpendicular to the longitudinal direction (L) of the blood sampling tube.

## Revendications

1. Séparateur (100) pour séparer du plasma sanguin et des cellules sanguines dans une canule de prélèvement sanguin (200), dans lequel le séparateur présente :
un corps flottant (110) ayant une première épaisseur et ayant au moins un bord d'étanchéité périphérique (116) ; et
un corps de ballast (120) ayant une deuxième épaisseur qui est plus grande que la première épaisseur ;
dans lequel la densité totale du corps flottant et du corps de ballast est située entre la densité du plasma et la densité des cellules dans le sang ;
dans lequel le corps flottant (110) et le corps de ballast (120) sont mobiles l'un par rapport à l'autre et forment un clapet ensemble ;
**caractérisé en ce que**
le corps flottant présente au moins une ouverture de passage (112) ;
que le bord d'étanchéité (116) du corps flottant (110) est conçu pour l'appui étanche périphérique contre l'intérieur de la canule ;
que pour former le clapet, le corps de ballast (120) présente en outre au moins un moyen de fermeture (122, 124) pour ouvrir ou fermer l'ouverture de passage (112) dans le corps flottant (110) ; et
qu'un élément de rappel (130) est prévu en tant que liaison élastique entre le corps flottant (110) et le corps de ballast (120) de façon à ce que pour ouvrir le clapet, une application de force soit nécessaire pour surmonter une force de rappel définie par l'élément de rappel (130).

2. Séparateur (110) selon la revendication 1, **caractérisé en ce que**
le moyen de fermeture (122) est conçu sous forme d'un poinçon qui peut être introduit dans l'ouverture de passage dans le corps flottant (110).

3. Séparateur (100) selon la revendication 1, **caractérisé en ce que**
le moyen de fermeture (124) est conçu sous forme d'une face d'appui en tant que zone surfacique sur le corps de ballast (120) pour un recouvrement étanche de l'ouverture de passage (112) dans le corps flottant (110) .

4. Séparateur (100) selon la revendication 1, **caractérisé en ce que**
l'élément de rappel (130) est fabriqué dans le même matériau que le corps flottant (110), formé de préférence d'une seule pièce avec celui-ci ou fabriqué dans le même matériau que le corps de ballast (120), formé de préférence d'une seule pièce avec celui-ci.

5. Séparateur (100) selon l'une des revendications précédentes,
**caractérisé en ce que**
le corps flottant (110) a la forme d'une trémie, dans lequel la trémie, partant d'une section d'ouverture de passage (114) contre laquelle le bord d'étanchéité (116) de préférence périphérique est formé pour l'appui étanche périphérique sur les faces intérieures des parois de la canule de prélèvement sanguin (200), se réduit en direction de l'au moins une ouverture de passage (112) et débouche dans l'ouverture de passage ; et
dans lequel la section d'ouverture de passage, rapporté à un alignement vertical du séparateur, est disposée au-dessus de l'ouverture de passage (112).

6. Séparateur (100) selon l'une des revendications précédentes,
**caractérisé en ce que**
le corps flottant (110) est fabriqué en matériau élastique, en particulier au niveau de son bord d'étanchéité (116).

7. Séparateur (100) selon l'une des revendications précédentes,
**caractérisé en ce que**
l'ouverture de passage (112) est ainsi alignée dans le corps flottant que la verticale sur le plan tendu par celle-ci présente avec l'axe principal (H) du séparateur, un angle de α = 45°, de préférence de α = 10°, plus encore de préférence de α = 0°.

8. Séparateur (100) selon l'une des revendications précédentes,
**caractérisé en ce que**
le corps flottant (110) présente au moins un étrier de retenue (118) pour retenir et/ou guider le corps de ballast (120), en particulier dans son mouvement par rapport au corps flottant (110).

9. Canule (200) comprenant un séparateur (100) en son intérieur,
**caractérisée en ce que**
le séparateur (100) est conçu selon l'une des revendications précédentes ; et
que le diamètre extérieur du corps flottant (110) formé par le bord d'étanchéité périphérique (116) est plus grand dans l'état détendu que le diamètre intérieur (d) de la canule de prélèvement sanguin (200) pour un appui étanche périphérique sur les faces intérieures des parois de la canule de prélèvement sanguin.

10. Procédé pour séparer du sang en plasma sanguin et cellules sanguines dans une canule de prélèvement sanguin (200) fermée à l'aide d'un séparateur (100) à l'intérieur de la canule de prélèvement sanguin selon l'une des revendications précédentes, comprenant les étapes suivantes :
- introduction de sang dans la canule (200), dans lequel le sang s'écoule dans l'intérieur de la canule en passant devant l'extérieur du séparateur reposant transversalement ;
- centrifugation de la canule avec le sang contenu à l'intérieur, dans lequel le plasma sanguin et les cellules sanguines se séparent, et dans lequel le séparateur (100) se détache d'une position de départ (A), passe dans un alignement axial par rapport à l'axe longitudinal (L) de la canule de prélèvement sanguin (200) et va dans une couche limite (G) entre le plasma séparé et les cellules ;
dans lequel le clapet dans le séparateur (100) est fermé dans la position de départ (A) ; et
dans lequel le clapet, sous l'action de la force centrifuge, s'ouvre contre une force de rappel, de façon à ce que les cellules du sang plus lourdes en comparaison du plasma, passent au travers du corps flottant (110) à travers l'ouverture de passage (112) ouverte pendant la centrifugation, sous l'effet de la force centrifuge, et se regroupent en-dessous du séparateur (100) dans la canule de prélèvement sanguin (200) tandis que le plasma plus léger reste au-dessus du séparateur dans la canule de prélèvement sanguin (200) .

11. Procédé selon la revendication 10,
**caractérisé en ce que**
le séparateur (100), après son détachement, va de la position de départ (A), en surmontant le frottement d'adhérence par la force centrifuge, dans la couche limite (G) ; et
que le bord d'étanchéité périphérique (116) du séparateur (100) repose sur l'intérieur de la canule de prélèvement sanguin (200) en étanchéité par friction et sur tout son pourtour avec le clapet après l'alignement axial du séparateur ; et
que le clapet se referme du fait de l'action d'une force de rappel, après son alignement axial dans la couche limite (G), après qu'il n'y a plus de force centrifuge.

12. Procédé selon la revendication 11,
**caractérisé en ce qu'**
à la suite de l'alignement axial du séparateur dans la couche limite (G) et après la fermeture du clapet, le plasma est prélevé de la canule de prélèvement sanguin, par exemple pipeté à l'aide d'une pipette ou par bascule.

13. Procédé selon l'une des revendications 10 à 12, **caractérisé en ce que**
le séparateur (100) est pré-monté dans la position de départ (A) avant l'introduction de sang dans la canule de prélèvement sanguin, dans lequel le séparateur (100) est aligné avec son axe principal (H) transversal à la direction longitudinale (L) de la canule de prélèvement sanguin.
